# EUROPÄISCHE PATENTSCHRIFT

(11) **EP 3 683 201 B1**
(45) Veröffentlichungstag und Bekanntmachung des Hinweises auf die Patenterteilung: **26.04.2023**
(21) Anmeldenummer: 19020034.5
(22) Anmeldetag: 18.01.2019
(51) Int. Cl.: C07C 1/20, C10G 3/00, C07C 11/06, C07C 4/06, C07C 7/00, C07C 7/09

(54) **VERFAHREN ZUR HERSTELLUNG VON OLEFINEN AUS OXYGENATEN**
PROCESS FOR PRODUCING OLEFINS FROM OXYGENATES
PROCÉDÉ DE PRODUCTION D'OLÉFINES À ARTIR DE COMPOSÉS OXYGÉNÉS

(43) Veröffentlichungstag der Anmeldung: 22.07.2020
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Ahlers, Bernd, 63128 Dietzenbach (DE); Castillo-Welter, Frank, 61381 Friedrichsdorf (DE); Drosdzol, Christopher, 60438 Frankfurt (DE); Gorny, Martin, 65760 Eschborn (DE); Haag, Stéphane, 60598 Frankfurt (DE); Janko, Lutz, 64390 Erzhausen (DE); Williams, Bryce, 60437 Frankfurt am Main (DE)
(74) Vertreter: Dropsch, Holger

(56) Entgegenhaltungen:
- DE-A1-102013 101 575

## Beschreibung

In einem Verfahren zur Herstellung von Olefinen aus Oxygenaten, umfassend die folgenden Schritte:
(i) Heterogen-katalysierte Umsetzung der Oxygenate in einem Oxygenat-zu-Olefin-Reaktor (OTO-Reaktor) unter Oxygenatumwandlungsbedingungen zu einem Produktstrom, der Wasser, Olefine und andere Kohlenwasserstoffe und wenigstens ein Oxygenat enthält,
(ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird,
   betrifft die Erfindung die weitere Aufarbeitung der erhaltenen Ströme zur Gewinnung insbesondere kurzkettiger Olefine wie Ethylen und Propylen.

### Stand der Technik

Kurzkettige Olefine, insbesondere Propylen (Propylen), gehören zu den wichtigsten Grundstoffen der chemischen Industrie. Dies liegt darin begründet, das ausgehend von diesen ungesättigten Verbindungen mit einer kurzen Kettenlänge Moleküle mit langkettigem Kohlenstoffgerüst und zusätzlichen Funktionalisierungen aufgebaut werden können.

Als Quelle für kurzkettige Olefine diente in der Vergangenheit vor allem das Steam-Cracking, d.h. die thermische Spaltung bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen) oder auch MTO- (Methanol-to-Olefin) Verfahren zur Herstellung von Propylen und anderen kurzkettigen Olefinen gehen von Methanol als Ausgangsstoff aus. Verallgemeinernd spricht man in diesem Zusammenhang auch von Oxygenat-zu-Olefin-(OTO)-Verfahren, da sauerstoffhaltige organische Komponenten wie Methanol oder Dimethylether (DME) auch als Oxygenate bezeichnet werden. In diesen heterogen katalysierten Verfahren wird demnach beispielsweise aus Methanol zunächst teilweise das Zwischenprodukt Dimethylether und nachfolgend aus einer Mischung von Methanol und Dimethylether eine Mischung aus Ethylen und Propylen sowie Kohlenwasserstoffen mit höherer Molmasse, darunter auch Olefine, gebildet. Zudem findet sich im Produktstrom Wasser, welches zum einen aus dem Prozessdampf stammt, der optional dem MTO-Reaktor zur Reaktionsmodulierung zugeführt wird und dem im MTP-Reaktor erzeugten Reaktionswasser.

Die sich anschließende Aufreinigung soll zum einen unerwünschte Nebenprodukte und unumgesetzte Edukte abtrennen und die einzelnen Kohlenwasserstofffraktionen möglichst rein darstellen. Üblicherweise wird hierzu im ersten Schritt ein Quenchsystem zur Anwendung gebracht. Unter Quenchen wird dabei eine schlagartige oder schockartige Abkühlung verstanden, die zumeist durch direkten Wärmeaustausch mit einem fluiden Quenchmedium bewirkt wird. Wenn hierzu eine Flüssigkeit wie Wasser oder Methanol verwendet wird, tritt zudem eine gewisse Reinigungswirkung in Bezug auf die verbliebene Gasphase auf.

An den Quenchschritt schließen sich zumeist eine Kompressionssektion und schließlich eine Aufarbeitungssektion an, bei der die verschiedenen Kohlenwasserstoffe als Fraktionen oder auch - insbesondere die als Zielprodukte begehrten Olefine - als isolierte Komponenten erhalten werden. Die Aufarbeitungssektion besteht dabei in der Regel aus einer Abfolge verschalteter Destillations- bzw. Rektifikationskolonnen.

Ein Beispiel für die einer OTO-Reaktion nachfolgende Aufreinigung findet sich in der DE 10 2014 112 792 A1, in der beschrieben wird, wie in einem ersten Schritt eine heterogen-katalysierte Umsetzung von wenigstens einem Oxygenat zu einem C2 Olefine, C3 Olefine, C4 Olefine, C5/6 Kohlenwasserstoffverbindungen und C7+ Kohlenwasserstoffverbindungen enthaltenden Produktstrom und in einem zweiten Schritt eine Abtrennung eines zu wenigstens 95 Gew.-% aus C3 Olefinen bestehenden Propylenstroms erzeugt wird.

Die Patentveröffentlichung DE 102013101575 A1 lehrt ein Verfahren zur Herstellung von Olefinen aus Oxygenaten, umfassend die folgenden Schritte: (i) heterogen-katalysierte Umsetzung von wenigstens einem Oxygenat zu einem flüssige und gasförmige organische Verbindungen und Wasser enthaltenden Gesamtstrom und (ii) Auftrennung des Gesamtstroms in einer ersten Trenneinrichtung in eine zu wenigstens 90 Vol.-% die gasförmigen organischen Verbindungen des Gesamtstroms enthaltende Fraktion, in eine zu wenigstens 90 Gew. -% die flüssigen organischen Verbindungen des Gesamtstroms enthaltende Fraktion und in eine zu wenigstens 90 Gew. -% das Wasser des Gesamtstroms enthaltende Fraktion.

Die weiteren Aufreinigungseinheiten, die in der DE 10 2014 112 792 A1 beschrieben werden, entsprechen dem im Stand der Technik üblichen Konzept. Durch das Quenchen kann bereits eine grobe Trennung der Fraktionen in Abhängigkeit von ihrer Kettenlänge der entstehenden Olefine aufgrund des teilweisen Auskondensierens erfolgen, so dass eine flüssige C4+ Fraktion aus dem Quench abgeführt werden kann. Die gasförmig abgetrennte C4- Fraktion wird anschließend in eine Kompressionsstufe eingespeist. Die aus der Kompression stammende C4- Fraktion wird dann einer Trennvorrichtung zugeführt, in der C3- Kohlenwasserstoffe von den C4+ Kohlenwasserstoffen separiert werden. In anschließenden Reinigungsschritten wird die C3 Fraktion in einer weiteren Trenneinrichtung von der C2- Fraktion getrennt, was aufgrund der geringen Siedepunkte der beiden Fraktionen unter Druck erfolgen muss. Insgesamt ist die Aufreinigung des Produktstroms kompliziert, da eine hohe Reinheit der Produkte angestrebt wird. Dies gilt für MTP-Anlagen mit üblichen Produktionskapazitäten von etwa 470 kta Propylen, wird aber noch entscheidender, wenn die Anlagenkapazität geringer ist (100 kta oder 200 kta Propylen). Dadurch sind kleine Anlagen zurzeit wirtschaftlich nicht rentabel.

Einer der Gründe für die hohen Investitionskosten solcher Anlagen ist die Verwendung von Propylen-Kältemittel als bevorzugtes Kühlmedium für die Kondensatoren der Destillationskolonnen. Für den Bau entsprechender Kältemittelsysteme sind zusätzliche Investitionen erforderlich. Die Verwendung von Propylen-Kältemittel erhöht zudem die Betriebskosten, da der Kältemittelverdichter von einem oder mehreren Elektromotoren oder Dampfturbinen angetrieben werden muss.

In der Vergangenheit gab es hierzu bereits Ansätze, dieses Problem zu lösen, die jedoch nicht zu einer deutlichen Verbesserung der Wirtschaftlichkeit des Verfahrens führten.

In Frerich J. Keil: Methanol zu Kohlenwasserstoffen: Prozesstechnologie (Microporous and Mesoporous Materials - Juni 1999) werden mehrere verwandte Prozesse beschrieben, bei denen das komplette Kohlenwasserstoffprodukt des OTO-Reaktors zunächst einem Demethanizer zugeführt wird, d. h. das komplette Kohlenwasserstoffprodukt des OTO-Reaktors in einem Trockner getrocknet werden musste. Diese komplette Trocknung ist kostenintensiv.

Sowohl US 7,332,639 als auch US 4,587,373 verwenden einen Debutanizer zur Abtrennung von C5+ Kohlenwasserstoffen, an den sich ein Depropanizer und ein Deethanizer zur Aufarbeitung des Kopfproduktes des Debutanizers anschließt.

Die US 8,309,776 B2, die US 7,678,958 B2 und die US 7,238,848 B2 beschreiben das klassische Kohlenwasserstofftrennungsschema von leichten Komponenten (Deethanizer, Depropanizer, Debutanizer). Dieses Konzept hat den Nachteil, dass das Trocknen und Kühlen des gesamten Kohlenwasserstoffstroms vor dem Eintritt in die erste Kolonne, die bei Temperaturen unterhalb des Gefrierpunktes von Wasser betrieben wird, erforderlich ist.

Zusammenfassend lässt sich sagen, dass die Wirtschaftlichkeit von OTO-Verfahren, insbesondere des MTP-Prozesses verbessert werden könnte, wenn durch eine Optimierung der Trennsequenz unter Beibehaltung der Trennleistung in der Aufarbeitungssektion Investitionen und Betriebskosten eingespart werden könnten.

### Beschreibung der Erfindung

Der Erfindung liegt daher die oben genannte Aufgabe zugrunde. Diese Aufgabe wird durch ein Verfahren mit den Merkmalen des Anspruchs 1 gelöst. Weitere, insbesondere vorteilhafte Ausgestaltungen finden sich in den jeweiligen abhängigen Ansprüchen.

### Erfindungsgemäßes Verfahren:

Verfahren zur Herstellung von Olefinen aus Oxygenaten, umfassend die folgenden Schritte:
(i) Heterogen-katalysierte Umsetzung der Oxygenate in einem Oxygenat-zu-Olefin-Reaktor (OTO-Reaktor) unter Oxygenatumwandlungsbedingungen zu einem Produktstrom, der Wasser, Olefine und andere Kohlenwasserstoffe und wenigstens ein Oxygenat enthält,
(ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird,
(iii) Auftrennen des Stroms G oder eines aus diesem resultierenden Strom und/oder des Stroms O oder eines aus diesem resultierenden Strom in einen Strom C4-, welcher zum überwiegenden Teil aus Kohlenwasserstoffen mit vier oder weniger Kohlenstoffatomen besteht und Olefine sowie Oxygenate umfasst, und in einen Strom C4+, welcher zum überwiegenden Teil aus Kohlenwasserstoffen mit vier oder mehr Kohlenstoffatomen besteht und Olefine umfasst,
(iv) Auftrennen des Stroms C4- in einen Strom C2-, der Ethylen enthält, und einen Strom C3+, der Propylen, Propan, C4 Kohlenwasserstoffe und Oxygenate umfasst,
(v) Auftrennen des Stroms C3+ in einen Strom C3, der der Propylen und Propan umfasst, und in einen Strom C4-O, der C4-Kohlenwasserstoffe und Oxygenate umfasst,
(vi) Auftrennen des Stroms C3 in einen Propylen umfassenden Strom und in einen Propan umfassenden Strom.

Die für die Umsetzung von Oxygenaten zu Olefinprodukten erforderlichen Oxygenatumwandlungsbedingungen sind dem Fachmann aus dem Stand der Technik, beispielsweise den eingangs erörterten Druckschriften, bekannt. Es sind diejenigen physikalisch-chemischen Bedingungen, unter denen ein messbarer, bevorzugt ein technisch relevanter Umsatz von Oxygenaten zu Olefinen erzielt wird. Notwendige Anpassungen dieser Bedingungen an die jeweiligen Betriebserfordernisse wird er auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Thermische Trennverfahren im Sinne der Erfindung sind alle Trennverfahren, die auf der Einstellung eines thermodynamischen Phasengleichgewichtes beruhen. Bevorzugt sind die die Destillation oder Rektifikation. Grundsätzlich ist aber auch der Einsatz anderer thermischer Trennverfahren denkbar, beispielsweise der Extraktion oder Extraktivdestillation.

Als Aufreinigungsschritt sind im Zusammenhang mit der vorliegenden Erfindung grundsätzlich alle Verfahrensschritte zu betrachten, die sich eines thermischen Trennverfahrens bedienen; bevorzugt wird die Destillation oder Rektifikation verwendet.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem an-deren der beiden Bereiche strömen kann, unbeachtlich etwaiger zwischengeschalteter Bereiche, Bauteile oder benötigter Fördermittel.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile, in Betracht ziehen, die ihm aufgrund seines Fachwissens zur Durchführung dieses Verfahrensschrittes notwendig oder sinnvoll erscheinen.

Unter Oxygenaten werden grundsätzlich alle sauerstoffhaltigen Kohlenwasserstoffverbindungen verstanden, die sich unter Oxygenatumsetzungsbedingungen zu Olefinen, insbesondere zu kurzkettigen Olefinen wie Propylen, und weiteren Kohlenwasserstoffprodukten umsetzen lassen.

Als kurzkettige Olefine werden im Rahmen der vorliegenden Erfindung insbesondere die Olefine verstanden, die bei Umgebungsbedingungen gasförmig vorliegen, beispielsweise Ethylen, Propylen sowie die isomeren Butene 1-Buten, cis-2-Buten, trans-2-Buten, iso-Buten.

Als höhere Kohlenwasserstoffe werden im Rahmen der vorliegenden Erfindung insbesondere diejenigen Kohlenwasserstoffe verstanden, die bei Umgebungsbedingungen flüssig vorliegen.

Zur Bezeichnung von Kohlenwasserstofffraktionen wird folgende Nomenklatur verwendet: "Cn Fraktion" bezeichnet eine Kohlenwasserstofffraktion, die überwiegend Kohlenwasserstoffe der C-Kettenlänge n, also mit n C-Atomen, enthält. "Cn-Fraktion" bezeichnet eine Kohlenwasserstofffraktion, die überwiegend Kohlenwasserstoffe der C-Kettenlänge n sowie mit niedrigeren C-Kettenlängen enthält. "Cn+ Fraktion" bezeichnet eine Kohlenwasserstofffraktion, die überwiegend Kohlenwasserstoffe der C-Kettenlänge n sowie mit höheren C-Kettenlängen enthält. Aufgrund der verwendeten physikalischen Trennverfahren, beispielsweise der Destillation, ist die Auftrennung hinsichtlich der C-Kettenlänge nicht in der Weise zu verstehen, dass Kohlenwasserstoffe mit anderer Kettenlänge rigoros ausgeschlossen werden. So enthält eine Cn- Fraktion je nach Verfahrensbedingungen des Trennverfahrens immer noch geringe Mengen von Kohlenwasserstoffen mit einer C-Zahl größer als n.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegende Anmeldung sind die Aggregatzustände gasförmig bzw. dampfförmig als synonym zu betrachten.

Unter einem Auftrennen eines Stoffstroms ist im Zusammenhang mit der vorliegenden Erfindung eine Aufteilung des Stroms in mindestens zwei Teilströme zu verstehen. Wenn nichts anderes angegeben wird, ist davon auszugehen, dass die stoffliche Zusammensetzung der Teilströme derjenigen des Ausgangsstroms entspricht, außer für die Fälle, bei denen dem Fachmann unmittelbar einsichtig ist, dass eine Änderung der stofflichen Zusammensetzung der Teilströme infolge der Bedingungen der Auftrennung zwangsweise auftreten muss.

Unter einer Benzinfraktion ist ein überwiegend, bevorzugt weitgehend vollständig aus höheren Kohlenwasserstoffen bestehendes, unter Umgebungsbedingungen flüssig vorliegendes Stoffgemisch zu verstehen, das geeignet sein kann, als Ottokraftstoff verwendet zu werden.

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern in konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Unter der Angabe "Strom X oder ein aus diesem resultierender Strom", wird verstanden, dass der resultierende Strom, häufig mit X' bezeichnet, aus dem Strom X durch einen oder mehrere physikalische und/oder chemische Behandlungs- oder Umwandlungsschritte hervorgegangen ist. Ein Beispiel für einen physikalischen Behandlungsschritt ist die Kompression.

Die Ausgestaltung der Aufarbeitungssektion insbesondere zur Gewinnung der kurzkettige Olefine, die stromabwärts des OTO-Reaktors, der Quenchstufe und einer optional vorhandenen Kompressionsstufe angeordnet ist, ist grundsätzlich für jede Art von Oxygenat zu Olefinanlage (OTO-Anlage) anwendbar, unabhängig von der Anlagengröße sowie vom spezifischen Design oder Konzept der vorgeschalteten Reaktionssektion, der Quenchsektion und der optional vorhandenen Kompressionssektion. Dabei werden gegenüber der aus dem Stand der Technik bekannten Aufarbeitung eines OTO-Reaktionsproduktes folgende Maßnahmen durchgeführt und folgende Vorteile erzielt:
Eine Minimierung des Kältemittelverbrauchs wird erreicht, indem der Deethanizer stromaufwärts vor dem Depropanizer angeordnet wird. In dieser Kolonnenkonstellation ist das Kopfprodukt des Depropanizers weitgehend frei von C2- Kohlenwasserstoffen. Dadurch ist die Kondensationstemperatur für das nahezu reine C3 Kopfprodukt höher als aus dem Stand der Technik bekannt und der Depropanizer-Kopfkondensator kann mit Kühlwasser anstatt mit C3 Kältemittel betrieben werden. Der C3 Kältemittelverbrauch wird somit deutlich reduziert, da der Depropanizer- Kopfkondensator der mit Abstand größte Verbraucher von Kältemittel in der OTO-Anlage. Die Investitionskosten und die Betriebskosten der OTO-Anlage werden hierdurch deutlich reduziert, vor allem weil die Größe des Kältemittelsystems reduziert werden kann.

Zu beachten ist hierbei jedoch, dass der Feedstrom zum Deethanizer vorzugsweise getrocknet werden sollte, da der Deethanizer mit Kältemittel bei Temperaturen unter dem Gefrierpunkt von Wasser betrieben wird. Die Trockner werden vorzugsweise bei niedrigen Temperaturen betrieben. Bei diesen Temperaturen und beim Betriebsdruck des Deethanizers befinden sich die C4+ Kohlenwasserstoffe in der flüssigen Phase. Aufgrund der Anordnung des Deethanizers stromaufwärts des Depropanzers ist es daher vorteilhaft, sowohl den Kohlenwasserstoff-Flüssigkeitsstrom als auch den Kohlenwasserstoffdampfstrom zu trocknen.

Durch das erfindungsgemäße Auftrennen insbesondere des Stroms O oder des nach optionaler Verdichtung erhaltenen Stroms O' im Debutanizer in einen Kopfprodukt-Strom C4-, welcher zum überwiegenden Teil aus Kohlenwasserstoffen mit vier oder weniger Kohlenstoffatomen besteht und Olefine sowie Oxygenate umfasst, und dem bevorzugten Trocknen des Stroms C4- bzw. der aus diesem nach einem Abkühlen erhaltenen flüssigen und dampfförmigen Teilströme vor dem Zuführen zum Deethanizer wird gewährleistet, dass der Anteil an schwereren Kohlenwasserstoffen der Fraktion C4+ in dem zu trocknenden Strom minimiert wird und sich somit die Baugröße des oder der Trockner verringert.

Im Unterschied zu der Erfindung wurde bislang in einem Verfahren gemäß Stand der Technik in dem Deethanizer ein C2- Strom, der über Kopf gewonnen wurde, von der die C3- Kohlenwasserstofffraktion abgetrennt, die zuvor als Kopfprodukt eines Depropanizers erhalten wurde. Druck und Temperatur im Deethanizer mussten dabei auf das verfügbare Kältemittel im Deethanizer-Kondensator eingestellt werden. Eine Möglichkeit, dies sicherzustellen, bestand darin, den Debutanizer, Depropanizer und Deethanizer bei ca. 20 bar zu betreiben und ein Kältemedium im Deethanizer-Kondensator zu verwenden, das wesentlich kälter ist als ein C3 Kältemittel (< - 40 °C). Eine zweite Möglichkeit bestand darin, den Depropanizer und Deethanizer oder auch den Deethanizer allein mit einem Druck zu betreiben, der den Einsatz von C3 Kältemittel im Deethanizer-Kondensator ermöglicht. Eine dritte Möglichkeit bestand darin, bevorzugt alle vorhandenen Kolonnen, jedenfalls aber die dem Deethanizer vorgeschalteten Kolonnen mit ca. 20 bar zu betreiben und nur einen Kompressor für den Kopfstrom des Deethanizers zu verwenden, um C3 Kältemittel als Betriebsmittel im Deethanizer-Kondensator verwenden zu können.

Im Ergebnis sorgt in der erfindungsgemäßen Ausgestaltung der Debutanizer (Schritt (i)) zuerst für die Trennung von schweren Kohlenwasserstoffen und der sich anschließende Deethanizer (Schritt (iii)) ermöglicht die Trennung von leichtersiedenden Komponenten. Der sich an diese beiden Kolonnen anschließende Depropanizer (Schritt (v)) benötigt dann kein Kältemittel als Betriebsmittel. Dies gibt folgende Vorteile:
1. Die Anordnung des Debutanizers stromaufwärts des Deethanizers gewährleistet die Abtrennung schwerer C4+ Kohlenwasserstoffe, um somit die Notwendigkeit der Trocknung dieser Kohlenwasserstoffe zu vermeiden.
2. Der Trocknungsaufwand und die benötigte Kühlleistung zur Konditionierung des Feedstroms für diejenige Trennkolonne, die zuerst bei Temperaturen unter dem Gefrierpunkt von Wasser betrieben wird (hier also der Deethanizer), wird minimiert.
3. Der Feedstrom zum Depropanizer, der als Sumpfprodukt des Deethanizers erhalten wird, besteht hauptsächlich aus C3- und einem kleinen Teil an C4 Kohlenwasserstoffen. Unter diesen Bedingungen kann der Depropanizer mit Kühlwasser betrieben werden. Es wird hierbei kein C3 Kältemittel benötigt.
4. Optional kann der Betriebsdruck des Depropanizers noch weiter erhöht werden, da sein Feedstrom nur aus einer flüssigen Phase besteht und somit eine Druckerhöhung auf einfache Weise mittels einer Pumpe erfolgen kann.
5. Ein optional vorhandener Wäscher zur Entfernung von Kohlendioxid aus dem Deethanizer-Kopfprodukt kann durch eine einfache Flashstufe ersetzt werden, wenn mindestens 40 % des CO₂-haltigen Deethanizer-Kopfproduktstroms als Purgestrom bzw. Spülstrom aus dem Verfahren entfernt werden.

Durch die erfindungsgemäße Ausgestaltung der Aufarbeitungssektion werden deutliche Einsparungen hinsichtlich der Investitionskosten und die Betriebskosten der OTO-Anlage erzielt, da wie beschrieben eine signifikante Größenreduzierung einzelner Anlagenkomponenten und/oder des Verbrauchs spezifischer, kostenintensiver Betriebsmittel ermöglicht wird.

### Weitere bevorzugte Ausgestaltungen der Erfindung

In einer besonderen Ausgestaltung der Erfindung werden Strom G und/oder Strom O einem bevorzugt mehrstufigen Kompressionsschritt zugeführt und es wird bei diesem Kompressionsschritt ein gasförmige Kohlenwasserstoffe und Olefine umfassender Strom G', ein flüssiger, zum überwiegenden Teil aus Wasser und Oxygenaten bestehender wässriger Strom W' und ein flüssiger, zum überwiegenden Teil aus Kohlenwasserstoffen bestehender und Olefine umfassender Strom O' erhalten. Hierdurch wird der Druck in den nachgeschalteten Trennkolonnen auf einen günstigen Wert, beispielsweise 15 bis 25 bara (bar, absolut), bevorzugt 20 bara eingestellt, so dass die Kopfkondensatoren der Trennkolonnen mit gängigen Kühlmitteln, beispielsweise C3 Kältemittel oder Kühlwasser, betrieben werden können.

Besonders bevorzugt ist es dabei, dass der aus Strom O erhaltene Strom O' aufgetrennt wird in einen Strom C4-, welcher zum überwiegenden Teil aus Kohlenwasserstoffen mit vier oder weniger Kohlenstoffatomen besteht und Olefine sowie Oxygenate umfasst, und einen Strom C4+, welcher zum überwiegenden Teil aus Kohlenwasserstoffen mit vier oder mehr Kohlenstoffatomen besteht und Olefine umfasst. Auf diese Weise werden die nachfolgenden Trennkolonnen mit einem Kohlenwasserstoffgemisch mit eingeschränktem C-Zahl-Bereich beaufschlagt, so dass dort die Trennung leichter, also mit weniger Trennböden und/oder eingeschränkterem Temperaturprofil erfolgen kann.

Bevorzugt wird dabei Strom G' mit Strom C4- zusammengeführt wird, so dass auch Wertstoffe, also insbesondere kurzkettige Olefine wie Ethylen und Propylen, die sich in der Gasphase befinden, den nachgeschalteten Trennkolonnen zugeführt und schließlich als Wertprodukte gewonnen werden können.

In einem Aspekt der Erfindung wird der Strom C4- oder der durch Zusammenführen von Strom G' mit Strom C4- erhaltene Strom dem Schritt (iv), also dem Deethanizer, zugeführt. Auf diese Weise werden die nachfolgenden Trennkolonnen mit einem Kohlenwasserstoffgemisch mit eingeschränktem C-Zahl-Bereich beaufschlagt, so dass dort die Trennung leichter, also mit weniger Trennböden und/oder eingeschränkterem Temperaturprofil erfolgen kann.

Bevorzugt wird es dabei, wenn der Strom C4- oder der durch Zusammenführen von Strom G' mit Strom C4- erhaltene Strom vor dem Zuführen zu Schritt (iv) abgekühlt wird, wobei ein gasförmiger Strom und ein flüssiger Strom erhalten wird, wobei der gasförmige Strom und der flüssige Strom vor dem Zuführen zu Schritt (iv) getrocknet werden. Durch diese Maßnahme werden zwei Ströme definierter Phase erhalten und eine Zweiphasenströmung vermieden, die sich durch Fluktuationen physikalischer Zustandsgrößen wie Druck oder Temperatur auszeichnet und sich daher hinsichtlich ihres Mengenstroms nur schwer regulieren lässt. Die Trocknung beider erhaltener Phasen vor Einleiten in den Deethanizer ist vorteilhaft, da es aufgrund der dort herrschenden tiefen Temperaturen ansonsten zur Eisbildung kommen könnte, die zu Verstopfungen der Leitungen und Apparate führt.

In einem weiteren Aspekt der Erfindung erfolgt das Auftrennen in den Schritten (iii), (iv), (v), (vi) mittels eines thermischen Trennverfahrens, bevorzugt mittels Destillation, fraktionierter Destillation, Rektifikation oder Extraktivdestillation oder Kombinationen daraus.

Besonders vorteilhaft ist es, wenn das Auftrennen in Schritt (v) mittels Extraktivdestillation erfolgt, wobei ein Oxygenat als Extraktionsmittel zugegeben wird. Auf diese Weise ist insbesondere eine effektive und trennscharfe Abtrennung der Oxygenate, beispielsweise des nicht umgesetzten Dimethylethers oder Methanols, möglich. Vorteilhaft wird dabei Methanol als Oxygenat-Extraktionsmittel zugegeben, dass eine gute Wirkung als Extraktionsmittel aufweist und einen prozesseigenen Stoff darstellt, so dass verfahrensfremde Stoffe als Extraktionsmittel vermieden werden. Vorteilhaft ist es dabei, dass mit der Erfindung der Kopfkondensator des Depropanizers, Schritt (v), mit Kühlwasser betrieben werden kann.

Die Bereitstellung eines C3 Kältemittels ist aufwendig, da spezielle Bereitstellungsanlagen wie Kühlvorrichtungen und Kompressoren erforderlich sind und der Energieaufwand beträchtlich ist. Deshalb ist es vorteilhaft, dass mit der Erfindung soweit wie möglich auf die Verwendung von C3 Kältemittel verzichtet und stattdessen Kühlwasser zur Kühlung der Kopfkondensatoren der Trennkolonnen verwendet werden kann.

Insbesondere ist es vorteilhaft, wenn das Auftrennen in den Schritten (iv) und (vi) mittels Rektifikation erfolgt, wobei als Kühlmedium in Schritt (iv) ein Propan und/ oder Propylen enthaltendes C3 Kältemittel und als Kühlmedium in Schritt (vi) Kühlwasser verwendet wird.

### Ausführungsbeispiel

Weitere Merkmale, Vorteile und Anwendungsmöglichkeiten der Erfindung ergeben sich auch aus der nachfolgenden Beschreibung der Zeichnung.

Es zeigen:

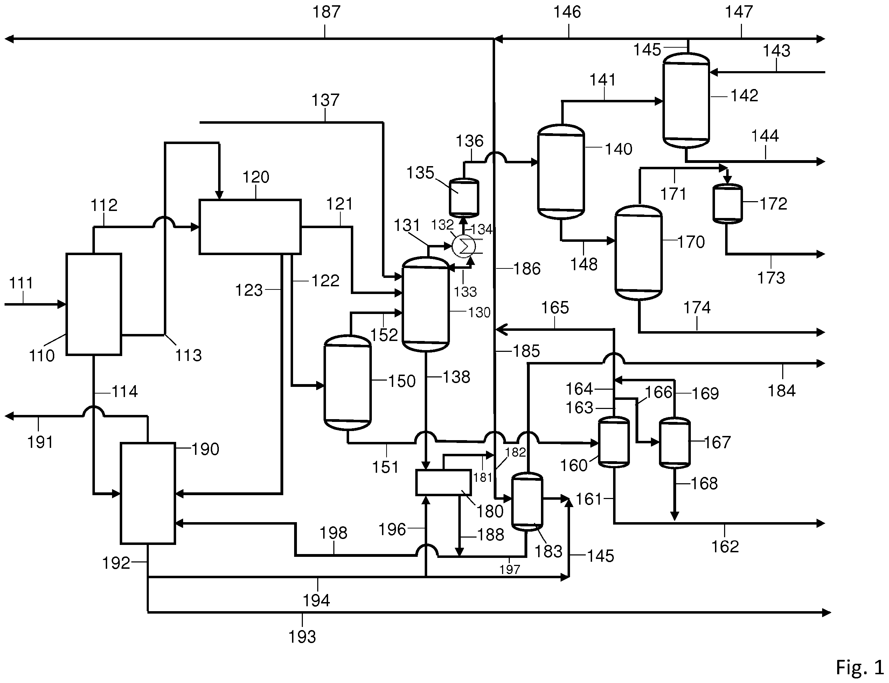

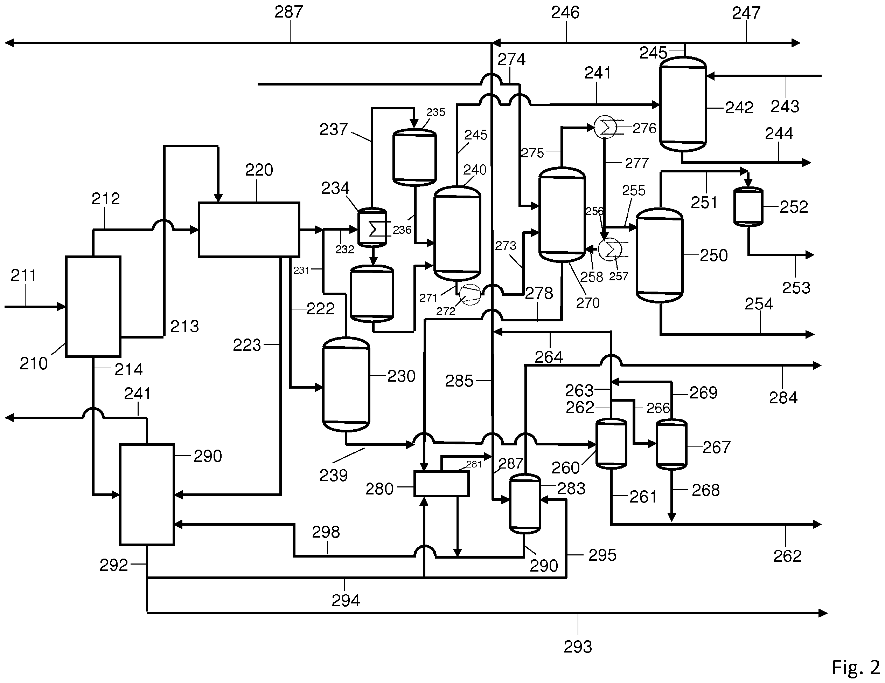

- Fig. 1: eine beispielhafte Ausgestaltung eines OTO-Verfahrens Fig. 2 gemäß dem Stand der Technik, eine erste beispielhafte Ausgestaltung eines OTO-Verfahrens gemäß der Erfindung.

Gemäß Figur 1 wird in einer aus dem Stand der Technik bekannten Anlage über Leitung 111 der Produktstrom aus einem OTO-Reaktor (nicht gezeigt) geführt, in welchem Oxygenate zu Olefinen umgesetzt wurden. Über diese Leitung 111 gelangt der Produktstrom in eine Quenchvorrichtung 110. Aus dieser wird zum einen eine gasförmige Phase über Leitung 112 in eine Kompressionsvorrichtung 120 überführt. Eine flüssige, ebenfalls im Wesentlichen aus organischen Komponenten bestehende, Phase wird über Leitung 113 ebenfalls in die Kompressionsvorrichtung eingebracht.

Eine wässrige Phase gelangt über Leitung 114 in ein nicht näher beschriebenes Methanolrückgewinnungssystem 190. Aus diesem werden über Leitung 191 Oxygenate zurück in den nicht dargestellten OTO-Reaktor recycelt. Über Leitung 192, 193 wird Wasser aus dieser Rückgewinnung ausgeschleust.

Aus der Kompressionsvorrichtung gelangt eine flüssige Phase über Leitung 122 in eine Trennkolonne 150, welche auch als Debutanizer bezeichnet wird. In dieser wird über Leitung 151 eine C4+ Fraktion abgezogen. Diese längerkettigen Olefine werden anschließend in eine weitere Trennkolonne 160 überführt, aus deren Sumpf über Leitung 161, 162 ein Benzinprodukt abgezogen wird. Über Leitung 163, 164, 165 wird ein C6- Strom in eine Recyclingleitung 186, 187 geleitet, die zum OTO-Reaktor zurückführt.

Aus Leitung 163 zweigt zudem die Leitung 166 ab, über welcher Teile dieses Stroms in eine Trennkolonne zur Benzinstabilisation 167 überführt werden können. Teile aus dieser Benzinstabilisation werden aus dem Sumpf über Leitung 168 dem Benzinproduktstrom zugemischt, während über Leitung 169 das Kopfprodukt in die Recyclingleitung 164, 165 überführt wird.

Über Leitung 152 gelangt das Kopfprodukt des sogenannten Debutanizers, der Trennkolonne 150, in die Trennkolonne 130, welche üblicherweise auch als Depropanizer bezeichnet wird. Dieser weist ein Rückflusssystem mit den Leitungen 131, 133 und dem Wärmetauscher 132 auf, von dem aus Teile des Kopfproduktes über Leitung 134 in nachgeschaltete Verfahrensschritte eingebracht werden. Bevorzugt ist die Trennkolonne 130 als Extraktivdestillation ausgestaltet, so dass über Leitung 137 ein Extraktionsmittel, beispielsweise Methanol, eingebracht wird, welches über Leitung 138 zusammen mit dem Sumpfprodukt aus der Trennkolonne 130 abgezogen wird.

Das über Leitung 134 ausgeschleuste Kopfprodukt der Trennkolonne 130 wird anschließend in eine Trocknungsvorrichtung 135 gebracht und von dort weiter über Leitung 136 in eine Trennkolonne 140 überführt. Aus diesem sogenannten Deethanizer wird über Leitung 141 das Kopfprodukt abgezogen und dieses in einen Wäscher 142 geführt. Diesem Wäscher wird über Leitung 143 Wasser als Waschmittel zugeführt und beladenes Waschmittel über eine Leitung 144 wieder abgeführt. Über die Leitung 145 wird das Kopfprodukt abgezogen, welches über Leitung 146 teilweise dem Recycling zugeführt wird, während es über Leitung 147 als C2- Produktstrom ausgeleitet werden kann. Aus diesem Strom kann Ethylen als Reinprodukt durch weitere, nicht gezeigte Aufarbeitung gewonnen werden.

Über Leitung 148 wird das Sumpfprodukt der Trennkolonne 140 in eine weitere Trennkolonne 170, als C3 Splitter bezeichnet, überführt. Als Sumpfprodukt wird hier die Propanfraktion abgetrennt, während das Kopfprodukt mittels der Leitung 171 der optionalen Vorrichtung 172 zugeführt und sodann über Leitung 173 das Wertprodukt Propylen abgezogen wird. Die optionale Vorrichtung 172 enthält beispielsweise ein Adsorptionsmittel zur Endreinigung des Propylenprodukts (z. B. zur Entfernung potentieller Katalysatorgifte wie DME-Spuren für eine nachgeschaltete, nicht gezeigte Polymerisierungsanlage).

Das Sumpfprodukt der Trennkolonne 130 wird über Leitung 138 in ein Mixer-Settler-System 180 geleitet. Aus diesem wird über Leitung 181 ein Strom abgezogen, welcher teilweise über Leitung 182 in eine Extraktionsvorrichtung 183 gelangt, aus welcher er über Leitung 184 ausgeschleust wird.

Ein anderer Teil wird über Leitung 185, 186 und 187 als Bestandteil des Gesamtreyclingstroms, welcher auch über die Leitung 164 und 165 gespeist wird, in den OTO-Reaktor zurückgeführt.

Über Leitung 188 wird ein Strom gewonnen, welcher mit einem aus der Extraktionsvorrichtung 183 stammenden wasser- und oxygenathaltigen Strom in Leitung 197 vermischt wird und über Leitung 198 in das Methanolrückgewinnungssystem 190 zurückgeführt wird.

Figur 2 zeigt im Vergleich dazu eine erfindungsgemäße Ausgestaltung. Ähnlich wie im Stand der Technik wird der Produktstrom des nicht dargestellten Reaktors über Leitung 211 in eine Quenchvorrichtung 210 geleitet, aus welcher über Leitung 212 eine dampfförmige organische Phase in eine Kompressionsvorrichtung 220 eingebracht wird. Über Leitung 213 wird in die Kompressionsvorrichtung 220 auch die flüssige organische Phase aus der Quenchvorrichtung 210 eingebracht.

Aus dieser Kompressionsvorrichtung 220 wird ein flüssiger Strom in die Trennvorrichtung 230 eingebracht, welche hier ebenfalls ein Debutanizer ist, also die C4+ Fraktion von der C4- Fraktion trennt. Über Leitung 231 wird das Kopfprodukt dieser Trennvorrichtung abgezogen und über eine Leitung 232 in eine Kühlvorrichtung 234 eingebracht. Dabei ist es möglich, jedoch nicht zwingend notwendig, dass über Leitung 233 entweder durch Zumischung in die Leitung 232 oder direkt in die Trocknungsvorrichtung 235 ein gasförmiger Strom aus der Kompressionsvorrichtung 220 in die Kühlvorrichtung 234 eingebracht wird.

Über Leitung 237 wird dann der gekühlte Strom in die Trocknungsvorrichtung 235 eingebracht, von wo aus er über Leitung 236 in die Trennkolonne 240 (Deethanizer) eingebracht wird. Die Trennkolonne 240 ist so ausgestaltet, dass über Kopf mittels Leitungen und 241 die C2- Fraktion abgezogen wird. Diese C2- Fraktion kann optional in einen Wäscher 242 eingebracht werden, welcher über 243 mit Wasser als Waschmittel gespeist wird, welches über Leitung 244 wieder abgezogen wird. Über Leitung 245 wird das Kopfprodukt dieses Wäschers entweder in Leitung 247 eingebracht, aus der es aus dem Prozess ausgeschleust wird, und/ oder über Leitung 246 dem Recyclingstrom in den nicht dargestellten OTO-Reaktor zurückgeführt.

Aus der Trennkolonne 240 wird das Sumpfprodukt über Leitung 271, Verdichter 272 und Leitung 273 in die Trennkolonne (Depropanizer) 270 eingebracht. In dieser wird die C3- Fraktion von höheren Kohlenwasserstoffen mittels Extraktivdestillation mit Methanol als Extraktionsmittel getrennt, das über Leitung 274 zugeführt wird. Die höheren Kohlenwasserstoffe werden über Leitung 278 in das Mixer-Settler-System 280 eingebracht. Über Leitung 275 wird das Kopfprodukt über den Wärmetauscher 276 und die Leitung 277 sowie 255 in die Trennkolonne (C3 Splitter) 250 eingebracht. Aus dieser Trennkolonne 250 wird das Wertprodukt Propylen über Kopf mittels Leitung 251 über Vorrichtung 252 und Leitung 253 abgezogen. Das Propan enthaltende Sumpfprodukt wird über Leitung 254 abgezogen. Die optionale Vorrichtung 252 enthält beispielsweise ein Adsorptionsmittel zur Endreinigung des Propylenprodukts (z. B. zur Entfernung potentieller Katalysatorgifte wie DME-Spuren für eine nachgeschaltete, nicht gezeigte Polymerisierungsanlage).

Die weitere Aufbereitung der oxygenathaltigen Wasser- und Kohlenwasserstoffstöme (z. B. in Leitung 278) entspricht derjenigen in Figur 1 (dort Leitung 138). Dasselbe gilt für die weitere Aufarbeitung des Sumpfproduktes der ersten Trennkolonne (Debutanizer), Leitung 151 in Fig. 1 bzw. Leitung 239 in Fig. 2. In diesem Zusammenhang verwendete Bezugszeichen mit gleicher Zehner- und Einerstelle haben eine analoge Bedeutung wie diejenigen gemäß Stand der Technik, Fig. 1.

### Bezugszeichenliste

- 110: Quenchvorrichtung
- 111-114: Leitung
- 120: Kompressionsvorrichtung
- 121-123: Leitung
- 130: Trennkolonne (Depropanizer)
- 131: Leitung
- 132: Wärmetauscher
- 133: Leitung
- 134: Leitung
- 135: Trocknungsvorrichtung
- 136: Leitung
- 140: Trennkolonne (Deethanizer)
- 141: Leitung
- 142: Wäscher
- 143-148: Leitung
- 150: Trennkolonne (Debutanizer)
- 151, 152: Leitung
- 160: Trennkolonne
- 161-166: Leitung
- 167: Trennkolonne (Benzinstabilisation)
- 168, 169: Leitung
- 170: Trennkolonne (C3 Splitter)
- 171: Leitung
- 172: Vorrichtung (Adsorber)
- 173, 174: Leitung
- 180: Mixer-Settler-System
- 181, 182: Leitung
- 183: Extraktionsvorrichtung
- 184-187: Leitung
- 190: Methanolrückgewinnungssystem
- 191-198: Leitung

- 210: Quenchvorrichtung
- 211-214: Leitung
- 220: Kompressionsvorrichtung
- 221-223: Leitung
- 230: Trennkolonne (Debutanizer)
- 231-233: Leitung
- 234: Kühler
- 235: Trocknungsvorrichtung
- 236-239: Leitung
- 240: Trennkolonne (Deethanizer)
- 241: Leitung
- 242: Wäscher
- 243-247: Leitung
- 250: Trennkolonne (C3 Splitter)
- 251: Leitung
- 252: Vorrichtung
- 253-256: Leitung
- 257: Wärmetauscher
- 258: Leitung
- 260: Trennkolonne
- 261-266: Leitung
- 267: Trennkolonne (Benzinstabilisation)
- 268, 269: Leitung
- 270: Trennkolonne (Depropanizer)
- 271: Leitung
- 272: Verdichter
- 273-275: Leitung
- 276: Wärmetauscher
- 277, 278: Leitung
- 280: Mixer-Settler-System
- 281, 282: Leitung
- 283: Extraktionsvorrichtung
- 284-287: Leitung
- 290: Methanolaufreinigungssystem
- 291-298: Leitung

## Patentansprüche

1. Verfahren zur Herstellung von Olefinen aus Oxygenaten, umfassend die folgenden Schritte:
(i) Heterogen-katalysierte Umsetzung der Oxygenate in einem Oxygenat-zu-Olefin-Reaktor (OTO-Reaktor) unter Oxygenatumwandlungsbedingungen zu einem Produktstrom, der Wasser, Olefine und andere Kohlenwasserstoffe und wenigstens ein Oxygenat enthält,
(ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird,
(iii) Auftrennen des Stroms G oder eines aus diesem resultierenden Strom und/oder des Stroms O oder eines aus diesem resultierenden Strom in einen Strom C4-, welcher zum überwiegenden Teil aus Kohlenwasserstoffen mit vier oder weniger Kohlenstoffatomen besteht und Olefine sowie Oxygenate umfasst, und in einen Strom C4+, welcher zum überwiegenden Teil aus Kohlenwasserstoffen mit vier oder mehr Kohlenstoffatomen besteht und Olefine umfasst,
(iv) Auftrennen des Stroms C4- in einen Strom C2-, der Ethylen enthält, und einen Strom C3+, der Propylen, Propan, C4 Kohlenwasserstoffe und Oxygenate umfasst,
(v) Auftrennen des Stroms C3+ in einen Strom C3, der der Propylen und Propan umfasst, und in einen Strom C4-O, der C4-Kohlenwasserstoffe und Oxygenate umfasst,
(vi) Auftrennen des Stroms C3 in einen Propylen umfassenden Strom und in einen Propan umfassenden Strom.

2. Verfahren nach Anspruch 1, **dadurch gekennzeichnet, dass** Strom G und/ oder Strom O einem bevorzugt mehrstufigen Kompressionsschritt zugeführt werden und bei diesem Kompressionsschritt ein gasförmige Kohlenwasserstoffe und Olefine umfassender Strom G', ein flüssiger, zum überwiegenden Teil aus Wasser und Oxygenaten bestehender wässriger Strom W' und ein flüssiger, zum überwiegenden Teil aus Kohlenwasserstoffen bestehender und Olefine umfassender Strom O' erhalten wird.

3. Verfahren nach Anspruch 2, **dadurch gekennzeichnet, dass** Strom O' aufgetrennt wird in einen Strom C4-, welcher zum überwiegenden Teil aus Kohlenwasserstoffen mit vier oder weniger Kohlenstoffatomen besteht und Olefine sowie Oxygenate umfasst, und einen Strom C4+, welcher zum überwiegenden Teil aus Kohlenwasserstoffen mit vier oder mehr Kohlenstoffatomen besteht und Olefine umfasst.

4. Verfahren nach Anspruch 3, **dadurch gekennzeichnet, dass** Strom G' mit Strom C4- zusammengeführt wird.

5. Verfahren nach Anspruch 1 oder 4, **dadurch gekennzeichnet, dass** Strom C4- oder der durch Zusammenführen von Strom G' mit Strom C4- erhaltene Strom dem Schritt (iv) zugeführt wird.

6. Verfahren nach Anspruch 5, **dadurch gekennzeichnet, dass** Strom C4- oder der durch Zusammenführen von Strom G' mit Strom C4- erhaltene Strom vor dem Zuführen zu Schritt (iv) abgekühlt wird, wobei ein gasförmiger Strom und ein flüssiger Strom erhalten wird, wobei der gasförmige Strom und der flüssige Strom vor dem Zuführen zu Schritt (iv) getrocknet werden.

7. Verfahren nach einem der vorigen Ansprüche, **dadurch gekennzeichnet, dass** das Auftrennen in den Schritten (iii), (iv), (v), (vi) mittels eines thermischen Trennverfahrens, bevorzugt mittels Destillation, fraktionierter Destillation, Rektifikation oder Extraktivdestillation oder Kombinationen daraus erfolgt.

8. Verfahren nach Anspruch 7, **dadurch gekennzeichnet, dass** das Auftrennen in Schritt (v) mittels Extraktivdestillation erfolgt, wobei ein Oxygenat als Extraktionsmittel zugegeben wird und wobei der Kopfkondensator des Depropanizers mit Kühlwasser betrieben wird.

9. Verfahren nach einem der vorigen Ansprüche, **dadurch gekennzeichnet, dass** das Auftrennen in den Schritten (iv) und (vi) mittels Rektifikation erfolgt, wobei als Kühlmedium in Schritt (iv) ein Propan und/oder Propylen enthaltendes Kältemittel und als Kühlmedium in Schritt (vi) Kühlwasser verwendet wird.

## Claims

1. Process for preparing olefins from oxygenates, comprising the following steps:
(i) heterogeneously catalysed conversion of the oxygenates in an oxygenate-to-olefin reactor (OTO reactor) under oxygenate conversion conditions to give a product stream containing water, olefins and other hydrocarbons and at least one oxygenate,
(ii) quenching the product stream in at least one quench stage to obtain a gaseous stream G comprising hydrocarbons and olefins, a liquid aqueous stream W consisting predominantly of water and oxygenates, and a liquid stream O consisting predominantly of hydrocarbons and comprising olefins,
(iii) separating stream G or a stream resulting therefrom and/or stream O or a stream resulting therefrom into a stream C4- consisting predominantly of hydrocarbons having four or fewer carbon atoms and comprising olefins and oxygenates, and into a stream C4+ consisting predominantly of hydrocarbons having four or more carbon atoms and comprising olefins,
(iv) separating the stream C4- into a stream C2- containing ethylene and a stream C3+ comprising propylene, propane, C4 hydrocarbons and oxygenates,
(v) separating the stream C3+ into a stream C3 comprising propylene and propane and into a stream C4-0 comprising C4 hydrocarbons and oxygenates,
(vi) separating the stream C3 into a stream comprising propylene and into a stream comprising propane.

2. Process according to Claim 1, **characterized in that** stream G and/or stream 0 is sent to a preferably multistage compression step and, in this compression step, a gaseous stream G' comprising hydrocarbons and olefins, a liquid aqueous stream W' consisting predominantly of water and oxygenates, and a liquid stream O' consisting predominantly of hydrocarbons and comprising olefins are obtained.

3. Process according to Claim 2, **characterized in that** stream O' is separated into a stream C4- consisting predominantly of hydrocarbons having four or fewer carbon atoms and comprising olefins and oxygenates, and a stream C4+ consisting predominantly of hydrocarbons having four or more carbon atoms and comprising olefins.

4. Process according to Claim 3, **characterized in that** stream G' is combined with stream C4-.

5. Process according to Claim 1 or 4, **characterized in that** stream C4- or the stream obtained by combining stream G' with stream C4- is sent to step (iv).

6. Process according to Claim 5, **characterized in that** stream C4- or the stream obtained by combining stream G' with stream C4- is cooled before being sent to step (iv), giving a gaseous stream and a liquid stream, where the gaseous stream and the liquid stream are dried before being sent to step (iv).

7. Process according to any of the preceding claims, **characterized in that** the separating in steps (iii), (iv), (v), (vi) is effected by means of a thermal separation process, preferably by means of distillation, fractional distillation, rectification or extractive distillation or combinations thereof.

8. Process according to Claim 7, **characterized in that** the separating in step (v) is effected by means of extractive distillation with addition of an oxygenate as extractant and with operation of the top condenser of the depropanizer with cooling water.

9. Process according to any of the preceding claims, **characterized in that** the separating in steps (iv) and (vi) is effected by means of rectification, using a propane- and/or propylene-containing coolant as cooling medium in step (iv) and using cooling water as cooling medium in step (vi).

## Revendications

1. Procédé pour la préparation d'oléfines à partir d'oxygénates, comprenant les étapes suivantes :
(i) transformation catalysée par voie hétérogène des oxygénates dans un réacteur oxygénate-en-oléfine (OTO) dans des conditions de transformation d'oxygénates en un flux produit qui contient de l'eau, des oléfines et d'autres hydrocarbures et au moins un oxygénate,
(ii) refroidissement brusque du flux produit dans au moins une étape de refroidissement brusque, suite à quoi on obtient un flux G gazeux comprenant des hydrocarbures et des oléfines, un flux W liquide, aqueux, constitué principalement d'eau et d'oxygénates et un flux O liquide, constitué principalement d'hydrocarbures et comprenant des oléfines,
(iii) séparation du flux G ou d'un flux résultant de celui-ci et/ou du flux O ou d'un flux résultant de celui-ci en un flux C4-, qui est constitué principalement d'hydrocarbures comprenant quatre atomes de carbone ou moins et qui comprend des oléfines ainsi que des oxygénates, et en un flux C4+, qui est constitué principalement d'hydrocarbures comprenant quatre atomes de carbone ou plus et qui comprend des oléfines,
(iv) séparation du flux C4- en un flux C2-, qui contient de l'éthylène, et en un flux C3+, qui comprend du propylène, du propane, des hydrocarbures en C4 et des oxygénâtes,
(v) séparation du flux C3+ en un flux C3, qui comprend du propylène et du propane, et en un flux C4-O, qui comprend des hydrocarbures en C4 et des oxygénates,
(vi) séparation du flux C3 en un flux comprenant du propylène et en un flux comprenant du propane.

2. Procédé selon la revendication 1, **caractérisé en ce que** le flux G et/ou le flux O est/sont introduit(s) dans une étape de compression de préférence à plusieurs étages et on obtient, dans cette étape de compression, un flux G' gazeux comprenant des hydrocarbures et des oléfines, un flux W' liquide, aqueux, constitué principalement d'eau et d'oxygénates et un flux 0' liquide, constitué principalement d'hydrocarbures et comprenant des oléfines.

3. Procédé selon la revendication 2, **caractérisé en ce que** le flux O' est séparé en un flux C4-, qui est constitué principalement d'hydrocarbures comprenant quatre atomes de carbone ou moins et qui comprend des oléfines ainsi que des oxygénates, et en un flux C4+, qui est constitué principalement d'hydrocarbures comprenant quatre atomes de carbone ou plus et qui comprend des oléfines.

4. Procédé selon la revendication 3, **caractérisé en ce que** le flux G' est rassemblé avec le flux C4-.

5. Procédé selon la revendication 1 ou 4, **caractérisé en ce que** le flux C4- ou le flux obtenu par rassemblement du flux G' avec le flux C4- est introduit dans l'étape (iv) .

6. Procédé selon la revendication 5, **caractérisé en ce que** le flux C4- ou le flux obtenu par rassemblement du flux G' avec le flux C4- est refroidi avant l'introduction dans l'étape (iv), un flux gazeux et un flux liquide étant obtenus, le flux gazeux et le flux liquide étant séchés avant l'introduction dans l'étape (iv) .

7. Procédé selon l'une quelconque des revendications précédentes, **caractérisé en ce que** la séparation dans les étapes (iii), (iv), (v), (vi) est réalisée au moyen d'un procédé de séparation thermique, de préférence au moyen d'une distillation, d'une distillation fractionnée, d'une rectification ou d'une distillation extractive ou d'une combinaison de celles-ci.

8. Procédé selon la revendication 7, **caractérisé en ce que** la séparation dans l'étape (v) a lieu au moyen d'une distillation extractive, un oxygénate étant ajouté en tant qu'agent d'extraction et le condensateur de tête du dépropaniseur fonctionnant avec de l'eau de refroidissement.

9. Procédé selon l'une quelconque des revendications précédentes, **caractérisé en ce que** la séparation dans les étapes (iv) et (vi) a lieu par rectification, un agent de refroidissement contenant du propane et/ou du propylène étant utilisé comme agent de refroidissement dans l'étape (iv) et de l'eau de refroidissement étant utilisée comme agent de refroidissement dans l'étape (v) .
